# EUROPEAN PATENT SPECIFICATION

(11) **EP 2 346 890 B1**
(45) Date of publication and mention of the grant of the patent: **20.03.2013**
(21) Application number: 08789129.7
(22) Date of filing: 05.09.2008
(51) Int. Cl.: C07H 19/06

(54) **ENANTIOMERIC RESOLUTION OF 2,4-DISUBSTITUTED 1,3-OXATHIOLANE NUCLEOSIDES**
TRENNUNG DER ENANTIOMEREN VON 2,4-DISUBSTITUIERTEN 1,3-OXATHIOLANNUKLEOSIDEN
RÉSOLUTION ÉNANTIOMÉRIQUE DE 1,3-OXATHIOLANE NUCLÉOSIDES 2,4-DISUBSTITUÉS

(43) Date of publication of application: 27.07.2011
(73) Proprietor: Shire Canada Inc., Ville Saint-Laurent, QC H4S 2C9 (CA)
(72) Inventor: VIZITIU, Dragos, Edmonton Alberta T6J 2C2 (CA); LACOSTE, Jean-Eric, Laval Quebec H7E 1L1 (CA); SIMION, Dan, deceased (CA)
(74) Representative: Heubeck, Christian
(86) International application number: PCT/IB2008/002314
(87) International publication number: WO 2010/026439

(56) References cited:
- EP-A- 1 153 924
- WO-A-95/08522
- US-A1- 2004 067 877
- US-A1- 2006 199 786

## Description

### FIELD OF THE INVENTION

The present invention relates to a novel process for producing (-) and (+) isomers of *cis* nucleosides or nucleoside analogues and derivatives of formula (A): 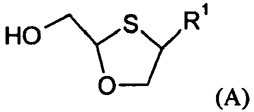 wherein R¹ is a pyrimidine base or a pharmaceutically acceptable derivative thereof.

### BACKGROUND OF THE INVENTION

Classes of compounds of formula (A), particularly the 2,4-disubstituted 1,3-oxathiolanes pyrimidine nucleosides and derivatives thereof, have been found to have potent antiviral activity. In particular, these compounds have been found to act as potent inhibitors of HIV-1 replication in T-lymphocytes over a prolonged period of time with less cytotoxic side effects than compounds known in the art (see Belleau et al (1993) Bioorg. Med. Chem. Lett. Vol. 3, No. 8, pp. 1723-1728). These compounds have also been found active against 3TC-resistant HIV strains (see Taylor et al (2000) Antiviral Chem. Chemother. Vol 11, No. 4, pp. 291-301; Stoddart et al (2000) Antimicrob. Agents Chemother. Vol. 44, No. 3, pp. 783-786). Additionally, the compounds of formula (A) are also useful in prophylaxis and treatment of hepatitis B virus infections.

Methods for the preparation of these compounds have been disclosed in PCT publications WO 92/08717, WO 95/29176 and WO 02/102796, as well as in publications by Belleau et al (1993) Bioorg. Med. Chem. Lett. Vol. 3, No. 8, pp. 1723-1728; Wang et al (1994) Tetrahedron Lett. Vol. 35, No.27, pp. 4739 -4742; Mansour et al , (1995) J. of Med. Chem. Vol. 38, No. 1, pp. 1-4 and Caputo et al in Eur. J. Org. Chem. Vol. 6, pp. 1455-1458 (1999).

The products of these processes are in many cases a racemate. These racemates require further processing to obtain the pure enantiomers. A preferred method for the production of single enantiomers is resolution of a racemate such as by direct preferential crystallization, crystallization of the diastereomeric salts, kinetic resolution, enzymatic resolution, selective absorption and asymmetric synthesis. See, e.g., EP 0 515 156, EP 0 515 157, EP 0560 794, EP 0 756 595, EP 0 757 684, EP 1 153 924, EP 1 361 227, EP 1 406 896, EP 1 473 294, EP 1 632 490, US 5,663,320, US 5,693,787, US 6,600,044, US 2006/0199786, WO 92/20669, WO 92/20696, and WO 2006/096954.

For example, Cimpoia et al. (US 2006/0199786) discloses a method preparing optically active cis-2-hydroxymethyl-4-(cytosine-1'-yl)-1,3-oxathiolane and derivatives thereof by reacting cis-oxathiolane compound with a chiral acid to for two diastereomeric salts, recovering one of the diastereomeric salts, and converting the recovered diastereomeric salt back into a enantiomer of the base compound.

If the racemate is a "true" racemic compound, a homogeneous solid phase of the two enantiomers co-exists in the same cell unit. These materials may be separated via diastereomer crystallization, which generally involves reacting the racemate with an optically pure acid or base (i.e., a resolving agent) to form a mixture of diastereomeric salts. These mixtures may be separated by preferential crystallization. However, some racemates may exist in the form of conglomerates. In a conglomerate, the individual enantiomers each crystallize as a single crystal lattice. Thus, a conglomerate salt is in effect a physical mixture of two separate crystal types, one of each isomer. But, conglomerates are typically observed in less than 20% of all racemates. See, e.g., Lorenz, H., et al., J. of the Univ. of Chem. Tech. and Metallurgy, (2007), 42 (1), 5-16 [5 to 10%of racemates belong to the conglomerate forming group].

A conglomerate can be defined as an equimolar mixture of two crystalline enantiomers that are, in principle, mechanically separable. The phase diagram of a conglomerate displays one sharply defined minimum temperature at a mixture of 50% and 50% that is the eutectic point of the enantiomeric mixture. The success of a preferential crystallization depends on this fact.

Methods for resolving certain racemates by formation of conglomerate salts, also known as preferential crystallization or resolution by entrainment, are described in, for example, Tung et al. (US 4,994,604), Manimaran et al. (US 5,302,751), and Coquerel et al. (US 6,022,409)

A conglomerate compound crystallizes as a single enantiomer in the crystal lattice, i.e., each crystal lattice is made up of a single enantiomer. Therefore, to be a conglomerate, the IR spectrum of the racemic conglomerate salt, a 1:1 mixture of (-) and (+) crystals, must be identical to that of the single enantiomer. Another characteristic of conglomerate behavior is that the racemic conglomerate salt normally has a melting point lower that that of either single enantiomer.

If a conglomerate is obtained, it may be used for enantiomeric excess enhancement because the most soluble composition is racemic. Generally, if the conglomerate has an excess of one enantiomer, that excess can be recovered, i.e., the conglomerate of X% enantiomeric excess will provide an X% yield of single enantiomer leaving behind racemic liquors.

A conglomerate in racemic form may also be used in an entrainment process in which a racemic solution is seeded with a single enantiomer leading to preferential kinetic precipitation of that enantiomer. See, e.g., Lorenz, H., et al., J. of the Univ. of Chem. Tech. and Metallurgy, (2007), 42 (1),5-16.

### Summary of the invention

While procedures as described above offer effective means to obtain single isomers of the *cis* nucleoside or nucleoside analogues and derivatives of formula (A): 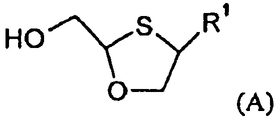 wherein R¹ is a pyrimidine base or a pharmaceutically acceptable derivative thereof, there is a need for a simpler and more economical process. The present invention is based on the discovery of a process which permits the enantiomers to be separated directly and efficiently by a direct crystallization technique using specific conglomerate salts.

US 2006/199786 teaches the preparation of optically active 3'-oxathiolan-derivatives, using an achiral acid.

EP 1 153 924 discloses the synthesis of single enantiomers of *cis*-substituted 1,3-oxathiolanes.

Thus, according to a process aspect of the present invention, there is provided a method for the preparation of single enantiomers of compounds of formula (B) in the cis configuration, and pharmaceutically acceptable salts and esters thereof, 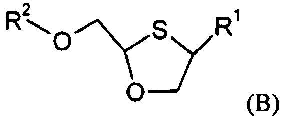 wherein
- R¹: is pyrimidine base or a pharmaceutically acceptable derivative thereof,
- R²: is hydrogen, a carboxyl function -C(O)-R³, or together with the oxygen atom to which it is attached forms an ester derived from a polyfunctional acid (such as phosphoric acids or carboxylic acids containing more than one carboxyl group, e.g., dicarboxylic acids of the formula HO₂C(CH₂)₁₋₁₀CO₂H); and
- R³: is selected from hydrogen, straight or branched chain (e.g., methyl, ethyl, n-propyl, t-butyl, n-butyl) or cyclic alkyl having 1 to 30 carbon atoms which is unsubstituted or substituted, alkoxyalkyl (e.g., methoxymethyl) having 2 to 30 carbon atoms which is unsubstituted or substituted, aralkyl (e.g., benzyl) having 7 to 18 carbon atoms which is unsubstituted or substituted, aryloxyalkyl (e.g., phenoxymethyl) having 7 to 18 carbon atoms which is unsubstituted or substituted, aryl having 6 to 14 carbon atoms which is unsubstituted or substituted (e.g., phenyl optionally substituted by halogen, C₁₋₄ alkyl or C₁₋₄ alkoxy), substituted dihydropyridinyl (e.g., N-methyldihydropyridinyl), sulphonate esters such as C₁₋₆-alkyl- or C₇₋₈-aralkylsulphonyl (e.g., methanesulphonyl), sulfate esters, amino acid esters (e.g., L-valyl or L-isoleucyl) and mono, di- or triphosphate esters,
**the process** comprising:
forming a conglomerate salt of a racemic mixture or an enantiomerically enriched mixture of a compound of formula (B) with an acid, wherein the resulting conglomerate salt has the following characteristics:
   the IR spectrum of the salt of the racemic compound, a 1:1 mixture of (-) and (+) crystals, is identical to that of each of the single enantiomer, and
   the salt of the racemic compound has a melting point lower that that of either single enantiomer;
obtaining an enantiomerically enriched mixture of the salts of the enantiomers by crystallization; and
obtaining the free base of the enantiomerically enriched mixture by standard methods (i.e., converting the salt into the free base).

Following formation of the conglomerate salt, the enantiomers can be separated by preferential crystallization such as described in Tung et al. (US 4,994,604), Manimaran et al. (US 5,302,751), and Coquerel et al. (US 6,022,409). The enantiomers may also be separated by a process of entrainment or cyclic entrainment.

In the present invention a solution of the *cis* nucleoside of formula B may be entrained by seeding with crystals of the desired single enantiomer to grow larger crystals having an excess of the isomer seeded, and leaving the opposite isomer enriched in the mother liquors. For an entrainment crystallization procedure to be useful for the production of single-enantiomer *cis* nucleoside of formula B, it is desirable that the enantiomerically enriched nucleoside obtained can be raised in enantiomeric purity through recrystallization or a series of recrystallizations. The mother liquors enriched with the opposite isomer may be treated further. The opposite isomer may be extracted via a similar recrystallization or it could be racemized, and the seeding process described above repeated allowing all the material in the mother liquor to be directed to the required *cis* isomer. Therefore, the process object of the present invention would provide crystals of higher enantiomeric excess (ee) of the desired isomer of the *cis* nucleoside of formula B. This would allow the present invention to be combined with methods which initially produce the *cis* nucleoside of formula B crystals of low ee, (such as a method of asymmetric synthesis that produces material of unacceptable ee) to provide a final product having a much higher ee of the desired product.

This process may also be used to prepare the single enantiomers of compounds of formula (B) in the *trans* configuration.

In an alternative embodiment of the present invention, the conglomerate salt of *cis* 2'-deoxy-3'-oxa-4'-thiocytidine is formed, wherein the single enantiomer shows a much lower solubility than the racemate in polar solvents.

The present invention includes the direct enantiomer separation of enantiomeric mixtures of *cis* 2'-deoxy-3'-oxa-4'-thiocytidine or *cis*/*trans* combinations of 2'-deoxy-3'-oxa-4'-thiocytidine without the need for resolving agents, by seeding a supersaturated solution of the 2'-deoxy-3'-oxa-4'-thiocytidine conglomerate salt with the desired single enantiomer 2'-deoxy-3'-oxa-4'-thiocytidine conglomerate salt, under controlled conditions.

The present invention also includes a process for the preparation of a single enantiomer of a compound of formula (B) or a pharmaceutically acceptable salt or ester thereof, wherein the enantiomer comprises methyl tosylate in an amount equal to or less than 2 ppm, the process comprising:
(a) forming a conglomerate salt of a racemic mixture or an enantiomerically enriched mixture of a compound of formula (B) with a tosic acid;
(b) obtaining an enantiomerically enriched mixture of the salts of the enantiomers by crystallization; and
(c) obtaining the free base of the enantiomerically enriched mixture, wherein the enantiomerically enriched mixture contains methyl tosylate in an amount equal to or less than 2 ppm.

### Detailed Description of the Invention

Accordingly, there is a provided in a first aspect of this invention the preparation of a single enantiomer of compounds of formula (B) in the cis configuration 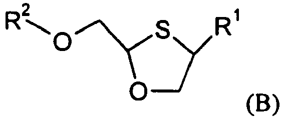 wherein R¹ and R² are as defined above, and pharmaceutically acceptable salts and esters thereof, via the formation of a conglomerate salt of a racemic mixture or an enantiomerically enriched mixture of a compound of formula (B) with an acid wherein the resulting conglomerate salt has the following characteristics: an IR spectrum of the salt of the racemic compound, a 1:1 mixture of (-) and (+) crystals, which is identical to each of the single enantiomer, and the salt of the racemic compound has a melting point lower that that of either single enantiomer.

In a preferred embodiment the single enantiomer further comprises a second isomer of a compound of formula (B) in an amount equal to or less than 1%. For example, in the case that the single enantiomer is the (-) *cis* isomer it will be understood that the second isomer may be selected from the (+) *cis* isomer, the (-) *trans* isomer, the (+) *trans* isomer and mixtures thereof.

The acid is preferably selected from maleic acid, achiral acids such as tosic acid, and mixtures thereof.

The present invention is based on the formation of a conglomerate salt of 2-substituted 4-substituted 1,3-oxathiolanes of formula (B) wherein R¹ is pyrimidine base or a pharmaceutically acceptable derivative thereof and R² is hydrogen, or together with the oxygen atom to which it is attached forms an ester of a polyfunctional acid, or a carboxyl function -C(O)-R³ in which the non-carbonyl moiety R³ of the ester grouping is selected from hydrogen, straight or branched chain alkyl (e.g., methyl, ethyl, n-propyl, t-butyl, n-butyl), C₃₋₈ cyclic alkyl, alkoxyalkyl (e.g., methoxymethyl), aralkyl (e.g., benzyl), aryloxyalkyl (e.g., phenoxymethyl), aryl (e.g., phenyl optionally substituted by halogen, C₁₋₄ alkyl or C₁₋₄ alkoxy); substituted dihydropyridinyl (e.g., N-methyldihydropyridinyl), sulphonate esters such as alkyl- or aralkylsulphonyl (e.g., methanesulphonyl), sulfate esters, amino acid esters (e.g., L-valyl or L-isoleucyl) and mono, di- or triphosphate esters.

Preferably, R¹ is selected from the following formulae: 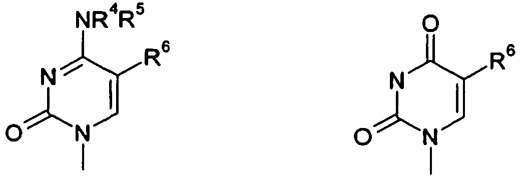 wherein
R⁴ and R⁵ are in each case independently H, straight, branched or cyclic C₁₋₆ alkyl, straight, branched or cyclic C₂₋₆ alkenyl, C₆₋₁₄ aryl, or C₅₋₁₀ heteroaromatic ring containing 1-3 heteroatoms wherein each heteroatom is O, N, or S heteroatoms; and
R⁶ is hydrogen, hydroxymethyl, trifluoromethyl, straight, branched or cyclic C₁₋₆ alkyl, straight, branched or cyclic C₂₋₆ alkenyl, bromine, chlorine, fluorine, or iodine.
R¹ may be, for example, cytosine or 5-fluorocytosine.
R² also includes esters derived from polyfunctional acids such as carboxylic acids containing more than one carboxyl group, for example, dicarboxylic acids HO₂C(CH₂)ₙCO₂H where n is an integer of I to 10 (for example, succinic acid) or phosphoric acids. For example, R² can be of the formula HO₂C(CH₂)ₙCO-O- where n is 1 to 10. Methods for preparing such esters are well known. See, for example, E. Hahn et al., "Nucleotide dimers as anti-human immunodeficiency virus agents", Nucleotide Analogues As Antiviral Agents, J. C. Martin, Ed. Symposium Series #401, American Chemical Society, pp. 156-159 (1989) and M. Busso et al., "Nucleotide dimers suppress HIV expression in vitro", AIDS Research and Human Retroviruses, 4(6), pp. 449-455 (1988).

The present invention includes the formation of a conglomerate salt of *cis* 2'-deoxy-3'-oxa-4'-thiocytidine wherein the single enantiomer shows a much lower solubility than the racemate in polar solvents. Direct enantiomer separation, without the need for resolving agents, can be achieved by seeding a supersaturated solution of the racemate with a single enantiomer, under controlled conditions. The separation may also be achieved for any derivative thereof.

An embodiment of the present invention includes a method for resolving *cis*-2-hydroxymethyl-4-(cytosin-1'-yl)-1,3-oxathiolane or derivatives or salts thereof comprising:
a) reacting said *cis*-2-hydroxymethyl-4-(cytosin-1'-yl)-1,3-oxathiolane with an achiral acid to produce *cis*-2-hydroxymethyl-4-(cytosin-1'-yl)-1,3-oxathiolane●achiral acid salt;
b) preparing a solution of *cis*-2-hydroxymethyl-4-(cytosin-1'-yl)-1,3-oxathiolane●achiral acid salt having an enantiomeric excess greater than zero;
c) adding to said solution an amount of (+) or (-)-*cis*-2-hydroxymethyl-4-(cytosin-1'-yl)-1,3-oxathiolane●achiral acid salt sufficient to initiate crystallization;
d) recovering substantially one of said (+) or (-)-*cis*-2-hydroxymethyl-4-(cytosin-1'-yl)-1,3-oxathiolane●achiral acid salt; and
e) converting said (+) or (-)-*cis*-2-hydroxymethyl-4-(cytosin-1'-yl)-1,3-oxathiolane●achiral acid salt into said (+) or (-)-*cis*-2-hydroxymethyl-4-(cytosin-1'-yl)-1,3-oxathiolane or salts.

Examples of achiral acids useful in the formation of conglomerate salts include hydrochloric acid (HCl), hydrobromic acid (HBr), sulfuric acid (H₂SO₄), tetrafluoroboric acid (HBF₄), methanesulfonic acid (CH₃SO₃H), benzenesulfonic(BS) acid (C₆H₅SO₃H), *p*-toluenesulfonic acid (*p-*CH₃C₆H₄SO₃H), *p*-aminoBS acid (*p*-NH₂C₆H₄SO₃H), *p*-chloroBS acid (*p*-ClC₆H₄SO₃H), *p-*hydroxyBS acid (*p*-HOC₆H₄SO₃H), chloroacetic acid (ClCH₂COOH), dichloroacetic acid (Cl₂CHCOOH), trichloroacetic acid (Cl₃CHCOOH), glycolic acid (HOCH₂COOH), pyruvic acid (CH₃COCOOH), succinic acid (HOOC(CH₂)₂COOH), adipic acid, (HOOC(CH₂)₄COOH), maleic acid (*Cis*-HOOCCH=CHCOOH),fumaric acid (*Tr*-HOOCCH=CHCOOH), citric acid (HOC(CO₂H)(CH₂CO₂H)₂), and mixtures thereof, among others.

In accordance with another aspect of the present invention there is provided a process for the preparation of a single enantiomer of a compound of formula (B) or a pharmaceutically acceptable salt or ester thereof, wherein the enantiomer comprises methyl tosylate in an amount equal to or less than 2 ppm, the process comprising the steps of:
(a) forming a conglomerate salt of a racemic mixture or an enantiomerically enriched mixture of a compound of formula (B) with a tosic acid;
(b) obtaining an enantiomerically enriched mixture of the salts of the enantiomers by crystallization; and
(c) obtaining the free base of the enantiomerically enriched mixture, wherein the enantiomerically enriched mixture contains methyl tosylate in an amount equal to or less than 2 ppm.

Preferably, the tosic acid is *para*-toluenesulfonic acid, the compound of formula (B) is 2'-deoxy-3'-oxa-4'-thiocytidine, and the enantiomer is in the *cis* configuration.

Described herein is also a composition comprising a single enantiomer of 2'-deoxy-3'-oxa-4'-thiocytidine or a pharmaceutically acceptable salt or ester thereof, wherein the enantiomer comprises methyl tosylate in an amount equal to or less than 2 ppm. Preferably, the enantiomer is in the *cis* configuration.

By the term " derivative" is a compound which is a pharmaceutically acceptable salt, ester, or salt of such ester of a compound of formula (B), or any other compound which, upon administration to the recipient, is capable of providing (directly or indirectly) a compound of formula (B) or an antivirally active metabolite or residue thereof. It will be appreciated by those skilled in the art that the compounds of formula (B) may be modified to provide pharmaceutically acceptable derivatives thereof, at functional groups in the base moiety.

The term "alkyl", as used herein, unless otherwise specified, refers to a saturated straight, branched, or cyclic, primary, secondary, or tertiary hydrocarbon of having 1-30 carbon atoms, preferably 1-6 carbon atoms, which is unsubstituted or optionally mono- or di-substituted by hydroxy, N₃, CN, SH, amino, halogen (F, Cl, Br, I), C₆-₁₂-aryl, C₁₋₆-alkyl, C₂₋₁₂-alkoxyalkyl, or nitro. It specifically includes methyl, ethyl, cyclopropyl, propyl, isopropyl, butyl, isobutyl, t-butyl, pentyl, cyclopentyl, isopentyl, neopentyl, hexyl, isohexyl, cyclohexyl, cyclohexylmethyl, 3-methylpentyl, 2,2-dimethylbutyl, and 2,3-dimethylbutyl.

Thus, R² can be, for example, methyl, ethyl, propyl, isopropyl, butyl, isobutyl, t-butyl, pentyl, isopentyl, neopentyl, hexyl, isohexyl, cyclopropyl, cyclobutyl, cyclopentyl, cyclohexyl, cyclopropylmethyl, cyclopentylmethyl, cyclohexylmethyl, 3-methylpentyl, 2,2-dimethylbutyl, 2,3-dimethylbutyl, halogenated C₁₋₆-alkyl, C₁₋₆-hydroxyalkyl, or C₁₋₆-aminoalkyl.

The term "alkenyl", as used herein, unless otherwise specified, represents an alkyl radical as defined herein wherein one or more -CH₂-CH₂- groups is in each case replaced by -CH=CH-. The alkenyl groups can be substituted in the manner described above for alkyl groups.

Alkoxyalkyl, as used herein, refers to alkyl-O-alkyl groups having up to 30 carbon atoms, preferably up to 6 carbon atoms, which in each case is unsubstituted or optionally mono- or di-substituted by hydroxy, N₃, CN, SH, amino, or halogen (F, Cl, Br, I). It specifically includes methoxymethyl, ethoxymethyl, propoxymethyl, and butoxymethyl.

The term "aryl" represents an aromatic moiety which is unsubstituted or substituted one or more times by hydroxy, N₃, CN, C₁₋₄ alkyl, C₁₋₄ alkoxy, and/or halogen (F, Cl, Br, I) and containing at least one benzenoid-type ring. The aryl groups contain from 6 to 14 carbon atoms (e.g., phenyl and naphthyl), particularly 6 to 10 carbon atoms.

The term "aralkyl" represents an aryl moiety which is attached to the adjacent atom by an alkyl group. The aryl portion of aralkyl is optionally substituted one or more times by hydroxy, N₃, CN, C₁₋₄ alkyl, C₁₋₄ alkoxy, and/or halogen (F, Cl, Br, I) and containing at least one benzenoid-type ring.

The term "aryloxyalkyl" represents an aryl moiety which is attached to an alkyl group by an oxygen atom, i.e., aryl-O-alkyl. The aryl portion of aryloxyalkyl is optionally substituted one or more times by hydroxy, N₃, CN, C₁₋₄ alkyl, C₁₋₄ alkoxy, and/or halogen (F, Cl, Br, I) and containing at least one benzenoid-type ring.

The term "protected" as used herein and unless otherwise defined refers to a group that is added to an oxygen, nitrogen, or phosphorus atom to prevent its further reaction or for other purposes. A wide variety of oxygen and nitrogen protecting groups are known to those skilled in the art of organic synthesis. Suitable protecting groups are described, for example, in Greene, et al., "Protective Groups in Organic Synthesis," John Wiley and Sons, Second Edition, 1991, hereby incorporated by reference.

According to an embodiment of the invention,
R¹ is selected from the following formulae: 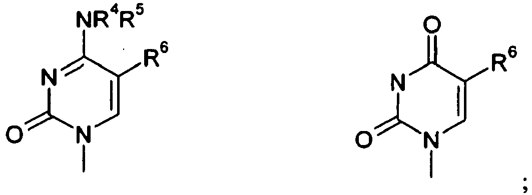 R² is -C(O)-R³;
R³ is methyl, ethyl, cyclopropyl, propyl, isopropyl, butyl, isobutyl, t-butyl, pentyl, cyclopentyl, isopentyl, neopentyl, hexyl, isohexyl, cyclohexyl, cyclohexylmethyl, 3-methylpentyl, 2,2-dimethylbutyl, 2,3-dimethylbutyl, methoxymethyl, phenyl, phenyl which is substituted by halogen, C₁₋₄₆ alkyl, or C₁₋₄ alkoxy, benzyl, or phenoxymethyl;
R⁴ and R⁵ are in each case independently H, straight, branched or cyclic C₁₋₆ alkyl, straight, branched or cyclic C₂₋₆ alkenyl, C₆₋₁₄ aryl, or C₅₋₁₀ heteroaromatic ring containing 1-3 O, N, or S heteroatoms; and
R⁶ is hydrogen, hydroxymethyl, trifluoromethyl, straight, branched or cyclic C₁₋₆ alkyl, straight, branched or cyclic C₂₋₆ alkenyl, bromine, chlorine, fluorine, or iodine.

According to another embodiment of the invention,
R¹ is cytosine or 5-fluorocytosine;
R² is -C(O)-R³; and
R³ is methyl, ethyl, cyclopropyl, propyl, isopropyl, butyl, isobutyl, t-butyl, pentyl, cyclopentyl, isopentyl, neopentyl, hexyl, isohexyl, cyclohexyl, cyclohexylmethyl, 3-methylpentyl, 2,2-dimethylbutyl, 2,3-dimethylbutyl, methoxymethyl, phenyl, phenyl which is substituted by halogen, C₁₋₄₆ alkyl, or C₁₋₄ alkoxy, benzyl, or phenoxymethyl.

The present invention includes the formation of crystalline *cis* 2'-deoxy-3'-oxa-4'-thiocytidine, enriched in the desired enantiomer, without requiring the use of seed crystals of desired enantiomer.

The present invention also includes the formation of crystalline *cis* 2'-deoxy-3'-oxa-4'-thiocytidine using a seed crystal of the desired enantiomer.

The present invention also includes the formation of crystalline *cis* 2'-deoxy-3'-oxa-4'-thiocytidine starting from *cis*/*trans* mixtures of 2'-deoxy-3'-oxa-4'-thiocytidine, wherein the *cis* to *trans* ratio is between about 1/1 to about 5/1.

The present invention also includes an entrainment process. Firstly, a saturated solution of the racemic *cis* 2'-deoxy-3'-oxa-4'-thiocytidine or a derivative is prepared at a given temperature. Of particular interest are solvents which favor the crystallization of the compound of formula (B). Suitable solvents include water, methanol, ethanol, toluene, tert-butyl methyl ether, isopropanol, n-propanol, acetone, and combinations thereof.

In an embodiment of the present invention, an amount of racemic mixture of *cis* 2'-doxy-3'-oxa-4'-thiocytidine or a derivative thereof is dissolved or suspended in a suitable solvent. Heat may be used to complete the dissolution. Concentrations above the saturation point may be used. The conglomerate is formed by adding in excess maleic acid, to the solution or suspension of the 2'-deoxy-3'-oxa-4'-thiocytidine or a derivative thereof to form a salt. The amount of achiral acid used is greater than about 1 eq. The amount of achiral acid may be between about 1 and about 3 eq. The conglomerate salt may be crystallized by conventional means. The melting point of the conglomerate salt is about 20 °C lower than the melting point of the single enantiomer salt. The eutectic point of the para-toluenesulfonic acid salt of 2'-deoxy-3'-oxa-4'-thiocytidine is between about 185 °C and 187°C The eutectic point of the maleic salt of 2'-deoxy-3'-oxa-4'-thiocytidine is between about 171 °C and 173°C.

Once the conglomerate salt is formed the reaction mixture may be seeded with crystals of the desired single enantiomer salt or the mixture may proceed to crystallization by conventional means. The seeded or unseeded mixture is then cooled and once crystallization has taken place, the precipitate product is harvested. The precipitate product shows a greater weight excess of desired single enantiomer salt. The mother liquor shows an excess of the enantiomer (opposite to that used for the seeding if seeding was used).

The precipitate product may be recrystallized by resuspending the precipitate product in a suitable recrystallization solvent. Suitable recrystallization solvents may include alcohols such as methanol, ethanol, isopropanol, acetone, and combinations thereof.

To obtain the free base, the precipitate product is resuspended in a suitable recrystallization solvent. Suitable recrystallization solvents include alcohols such as methanol, ethanol, isopropanol, acetone, and combinations thereof. If necessary, the pH is adjusted so that the mixture is basic (pH ≥ 7). A base is used to remove the acid. The base may be a free amine such as triethylamine, diethylcyclohexylamine, diethylmethylamine, dimethylethylamine, dimethylisopropylamine, dimethylbutylamine, dimethylcyclohexylamine, tributylamine, diethylmethylamine, dimethylisopropylamine, diisopropylethylamine or combinations thereof, or an immobilized base such as anion exchange resin or even ammonia. If a resin is used, the resin may be removed by filtration. The free base is cooled and the resulting precipitate is dried. The resultant crystalline *cis* 2'-deoxy-3'-oxa-4'-thiocytidine is enriched in the desired enantiomer. The amount of base added should be sufficient to remove all of the acid counter ions.

When a tosic acid such as *para*-toluene sulfonic acid is employed to form the conglomerate salt, the resultant enantiomer preferably comprises methyl tosylate in an amount equal to or less than 2 ppm.

The mother liquors resulting from the above described procedure contain an excess of one enantiomer that can be re-subjected to the above procedure by seeding with the opposite enantiomer. By an iterative process of crystallization (cyclic entrainment), seeding with opposite enantiomers alternately, it is, in principle, possible to separate an amount of racemic 2'-deoxy-3'-oxa-4'-thiocytidine entirely into its enantiomeric components.

In the enantiomeric enrichment (ee) procedure of this invention, the recrystallization may be preformed in a variety of solvents. These solvents can be chosen and the crystallization process induced by conventional techniques that lead to the formation of a supersaturated solution. Examples of such conventional techniques include cooling of a saturated solution, solvent evaporation from a saturated solution, or by employing a counter solvent in which the desired end product, such as *cis*-2'-deoxy-3'-oxa-4'-thiocytidine, is less soluble.

The present invention additionally includes the preparation of conglomerate salts described above using *cis*/*trans* mixtures of 2-substituted 4-substituted 1,3-oxathiolanes, wherein the *cis* to *trans* ratio (C/T) is between about 1/4 to about 4/1, for example, 1.6/1 to 3.5/1, especially 2/1 to 3/1.

In general, if an enantiomerically enriched mixture or a *cis*/*trans* combination (wherein C/T >1) of a compound of formula (B) is to be separated, the process may proceed through the following steps:1) formation of the conglomerate salt; 2) isolation of the enantiomerically enriched precipitate salt; 3) liberation of the enantiomerically enriched free base (the compound of formula (B)) from the precipitate salt by reaction of the salt with a proper base; 4) isolation of the enantiomerically enriched compound of formula (B) precipitate.

An embodiment of the present invention is a process for producing (-)-*cis*-2-hydroxymethyl-4-(cytosin-1'-yl)-1,3-oxathiolane, comprising:
a) preparing a solution of *cis*-2-hydroxymethyl-4-(cytosin-1'-yl)-1,3-oxathiolane●achiral acid salt having an enantiomeric excess different than zero;
b) crystallizing substantially (-)-*cis*-2-hydroxymethyl-4-(cytosin-1'-yl)-1,3-oxathiolane●achiral acid salt;
c) recovering said (-)-*cis*-2-hydroxymethyl-4-(cytosin-1'-yl)-1,3-oxathiolane●achiral acid salt;
d) converting said (-)-*cis*-2-hydroxymethyl-4-(cytosin-1'-yl)-1,3-oxathiolane●achiral acid salt into said (-)-*cis*-2-hydroxymethyl-4-(cytosin-1'-yl)-1,3-oxathiolane or pharmaceutically acceptable salts thereof.

Another embodiment of the present invention is the *para*-toluenesulfonic acid salt of 2'-deoxy-3'-oxa-4'-thiocytidine having an eutectic point between about 185 °C and 187°C.

Another embodiment of the present invention is the of the maleic salt of 2'-deoxy-3'-oxa-4'-thiocytidine having an eutectic point between about 171 °C and 173°C.

### Brief Description of the Drawings

Various features and attendant advantages of the present invention will be more fully appreciated as the same becomes better understood when considered in conjunction with the accompanying drawings, wherein:
Figure 1 is a phase diagram of the *p*-Toluenesulfonic acid salt of Compound (1); and
Figure 2 illustrates the UV and optical rotation monitoring of the crystallization process shown in Example 2.

### Examples

### Example 1

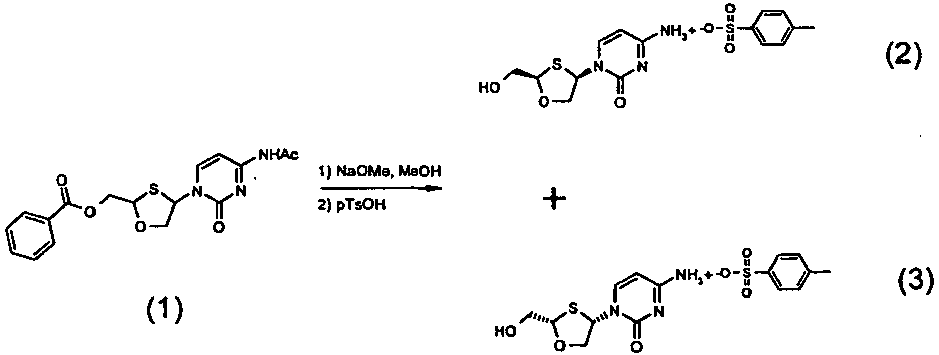

Compound (1) was prepared as described in PCT publication WO 02/102796. Sodium methoxide (0.1 eq.) was added in one portion to a methanol suspension (70 mL) of Compound 1 (1.0 eq.) at room temperature. The reaction mixture was stirred for 2 hrs at room temperature. TLC analysis (Hexane/EtOAc:1/9) showed the disappearance of starting material and the appearance of the more polar deprotected (1). *para*-Toluenesulfonic acid (1.14 eq.) was added to the solution in one portion at room temperature. The reaction mixture was allowed to stir at room temperature overnight. The reaction mixture was cooled to 0°C - 5°C. The suspension was stirred at this temperature for 1 hour then filtered. The solids were dried to give pure Compounds (2) and (3) as a white solid.

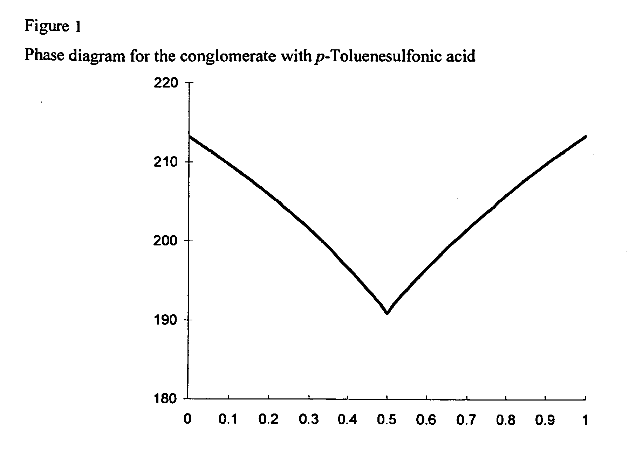

The *p*-TSA salts of both enantiomers and racemates were prepared and recrystallized from methanol/water/IPA. The maleic acid salt was obtained using the same solvent system. The IR spectra and Differential Scanning Calorimetry (DSC) results are shown in Table 1, Table 2 and Figure 1.

**Table 1**

| ***p*-TSA Salt** | **IR Match** | **DSC data (5°C/min)** | |
|---|---|---|---|
| | | **Melting Point (°C)** | **ΔH (J/g)** |
| Racemate | Yes | 186.8 | 100.2 |
| (-) enantiomer | Yes | 213.2 | 145.6 |
| (+) enantiomer | Yes | 214.2 | |

The maleic acid salt was prepared in a similar fashion.

**Table 2**

| **Maleic acid Salt** | **IR Match** | **DSC data (7 °C/min)** | |
|---|---|---|---|
| | | **Melting Point (°C)** | **ΔH (J/g)** |
| Racemate | Yes | 172.0 | - |
| Single enantiomer | Yes | 238.8 | - |

### Example 2

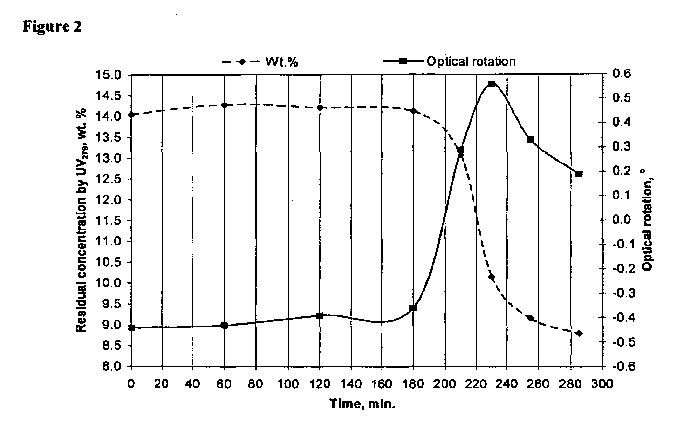

A 13 wt% racemate mixture of Compound (2) and (3) solution was prepared by dissolving 104.60 g of the racemate in 700 ml water. A 4% ee was generated by adding 4.36 g Compound (2) to the mixture. The solids were dissolved by heating the slurry at 50°C. The warm solution was cooled rapidly to 20°C and then, agitated at this temperature for 1 more hour to ensure its stability. Next, the supersaturated solution was seeded with 202 mg of finely ground Compound (2) (25 mg/100 g solution). The temperature was maintained constant at 20 ± 1°C with constant agitation. The course of crystallization was monitored by UV at 278 nm and polarimetry (see Figure 2 below).

The optical rotation of the starting supersaturated solution was - 0.44°. A 3-hour induction period was recorded before the crystallization occurred. Approximately 20 minutes into crystallization, the rotation of the solution dropped to zero. Further, the rotation changed sign and reached the maximum of + 0.56° after about 50 minutes of crystallization. Changing rotation sign of the supernatant solution indicates that entrainment and resolution has occurred. The solids were filtered out and the optical purity determined. The isolated solid had a higher optical purity than the initial supersaturated solution.

### Example 3

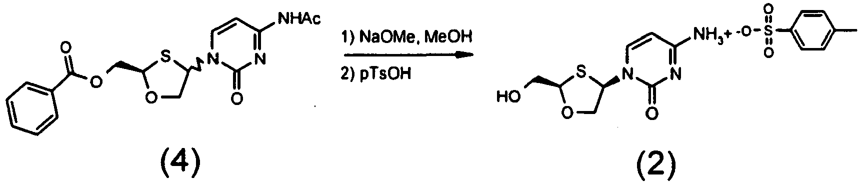

Sodium methoxide (0.1 eq.) was added in one portion to a methanol suspension of Compound (4) (1.0 eq, C/T=3.04/1, 95% ee, 96.6% purity) at room temperature. The reaction mixture was stirred for 2 hrs at room temperature. TLC analysis (Hexane/EtOAc: 1/9) showed the disappearance of starting material (Rf 0.10 (*trans*) and 0.16 (*cis*)) and the appearance of the more polar deprotected Compound (4) (Rf 0.00). The *para*-toluenesulfonic acid (1.14 eq.) was added to the solution in one portion at room temperature. The reaction mixture was allowed to stir at room temperature overnight then filtered. The solids were dried *in vacuo* to give Compound (2) salt as a white solid (62/1 *cis*/*trans:* 98% ee, 85% *cis* yield corrected).

### Example 4

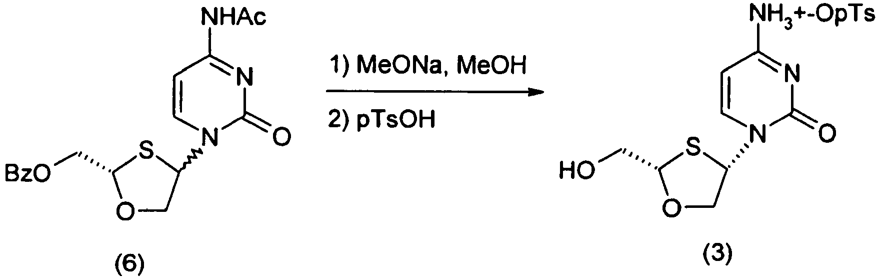

Sodium methoxide (0.1 eq.) was added in one portion to a methanol suspension of Compound (6) (1.0 eq., C/T=2.7/1, 95% ee, 96.0% purity) at room temperature. The reaction mixture was stirred for 2 hrs at room temperature. TLC analysis (Hexane/EtOAc: 1/9) showed the disappearance of starting material (Rf 0.10 (*trans*) and 0.16 (*cis*)) and the appearance of the more polar deprotected Compound (4) (Rf 0.00). *para*-Toluenesulfonic acid (1.13 eq.) was added to the solution in one portion at room temperature. The reaction mixture was allowed to stir at room temperature overnight then filtered. The precipitate was dried *in vacuo* to give Compound (3) salt as a white solid (62/1 *cis*/*trans:* 99% ee, 86% *cis* yield corrected).

### Example 5

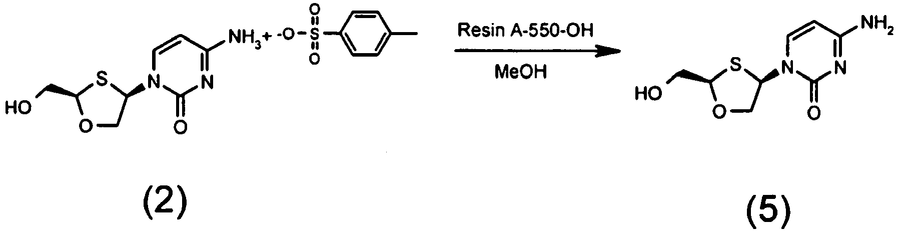

Compound (2) (9.76 mmoles, 1.0 eq.) was suspended in methanol at 40°C. Resin DOWEX 550A-OH (140%w/w) was added to the suspension in one portion at 40°C. The reaction mixture was allowed to stir at 40°C for 2 hrs. The pH of the solution was checked to make sure that it's basic (pH ≥ 7) and a sample was analyzed by ¹H NMR and showed the disappearance of *para*-toluenesulfonic acid. The reaction mixture was filtered. The resin was washed with methanol at 40°C. MeOH was distillated and the volume adjusted to 3 volumes. The solution was cooled and precipitation occurred. The suspension was stirred until no additional precipitation was observed then filtered. The solids were dried to give Compound (5) as a white solid (99.7 % ee, 82% *cis* yield).

### Example 6

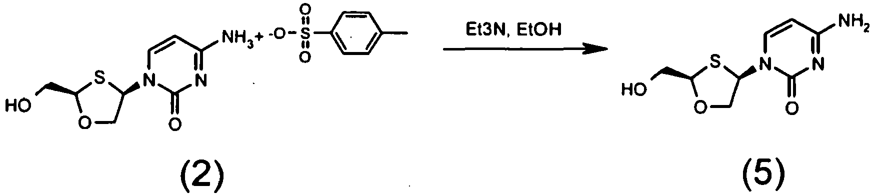

Compound (2) (99.1% ee, C/T= 27/1), 6.23 mmoles of *cis,* 1.0 eq.) was suspended in ethanol at 25°C. Triethylamine (9.33 mmoles, 1.5 eq.) was added to the suspension in one portion at 25 °C. The reaction mixture was heated at 40°C and stirred for 1 hr at this temperature. The pH of the solution was checked to make sure that it's basic (pH ≥ 7). The solution was cooled and precipitation occurred. The suspension was stirred until no additional precipitation was observed then filtered. The solids were washed with cold ethanol. The solids were dried to give Compound (5) as a white solid (99.4 % ee, 80% *cis* yield).

### Example 7

Compound (5) was analysed on a C18 column (length 15cm, diameter 4.6mm, particle size 3µm) using the following HPLC conditions:
Isocratic elution
Mobile Phase: 43% acetonitrile, 57% water + phosphoric acid 1/1000
Flow: 2 mL/min
Stop time: 10 min
Injection volume: 20 µL
Wavelength: 225 nm
Oven temperature: 40°C
The amount of methyl tosylate and *iso-*propyl tosylate detected (in ppm) in each batch of Compound (5) is shown in Table 3

**Table 3**

| **Item** | **Methyl tosylate** | **Isopropyl tosylate** |
|---|---|---|
| LOQ | 0.1628µg/ml | 0.2431µg/ml |
| | 1.6ppm | 2.4ppm |
| LOD | 0.0651µg/ml | 0.085µg/ml |
| | 0.65ppm | 0.85ppm |
| Batch 30-1600 | 0.1ppm | ND |
| Batch 30-1727 | 0.8ppm | ND |
| Batch 30-1739 | 0.4ppm | ND |
| Batch 30-1850 | 0.2ppm | ND |
| Batch 30-1875 | 0.5ppm | ND |
| Batch 30-2077 | 0.8ppm | ND |

| | | |
|---|---|---|
| LOQ is Limit of Quantification LOD is Limit of Detection | | |

### Example 8

### Screening for a conglomerate of cis 2'-deoxy-3'-oxa-4'-thiocytidine

The amine functionality of cis 2'-deoxy-3'-oxa-4'-thiocytidine was derivatized by salt formation with achiral acids. Four major groups of acids were screened to identify salts that may be candidates exhibiting conglomerate behavior:
● Inorganic acids (e.g.: HCl, HBr, H₂SO₄, HBF₄);
● Sulfonic (e.g.: methanesulfonic, benzenesulfonic, p-toluenesulfonic, p-hydroxytoluenesulfonic, sulfanilic, p-cholorobenezenesulfonic);
● Substituted acetic acids (e.g.: glycolitic, chloro-, dichloro-, trichloroacetic); and
● Polycarboxylic and oxy acids (e.g.: succinic, adipic, maleic, fumaric, citric, pyruvic).

In each case the salts of the racemic mixture and the single enantiomer were generated by reacting the nucleoside with an acid in water until the nucleoside was completely dissolved. The mixture was heated, if needed, until a clear solution was obtained. The salts were precipitated by vacuum concentration of the aqueous solution followed by the addition of isopropanol. The salt formation was confirmed ¹H NMR. In the case of HCl and HBr salts, a silver nitrate titration was performed. If a solid resulted an IR spectra was obtained.

A conglomerate compound crystallizes as a single enantiomer in the crystal lattice. This means that for a conglomerate compound the IR spectrum of the racemate (1:1 mixture of enantiomers) will be identical to that of the single enantiomer. Another characteristic of conglomerate behavior is that the racemate salt will have a melting point at least 25 °C lower than that of either single enantiomer salt.

For the screened salts, DSC data was obtained for each solid (see Table 4 below) and a binary melting point diagram was generated.

**Table 4**

| **Achiral Acid** | **Salt Formed?** | | **IR Match?** | **DSC data (°C)** | | **Possible Conglomerate Candidate?** |
|---|---|---|---|---|---|---|
| | **Racemate** | **Single Enantiomer** | | **Racemate** | **Single Enantiomer** | |
| HCl | YES | YES | NO | 137.8/229.2 | 139.1/221.5 | NO |
| HBr | YES | YES | NO | 228.8 | 217.9 | NO |
| H₂SO₄ | YES | YES | NO | 226.2 | 142.1/228.4 | NO |
| HBF₄ | YES | YES | NO | 150.5/211.5 | 218.3 | NO |
| Methanesulfonic | YES | YES | NO | 192.6 | 196.9 | NO |
| CH₃SO₃H | | | | | | |
| Benzenesulfonic (BS) | YES | YES | NO | 205.1 | 192.0 | NO |
| C₆H₅SO₃H | | | | | | |
| p-ChloroBS | YES | YES | NO | 126.8 | 146.6 | NO |
| P-ClC₆H₄SO₃H | | | | | | |
| p-Toluenesulfonic | YES | YES | YES | 185.2 | 214 | YES |
| p-CH₃C₆H₄SO₃H | | | | | | |
| p-AminoBS | YES | YES | YES | 224.0 | 227.1 | YES |
| p-NH₂C₆H₄SO₃H | | | | | | |
| Glycolic | YES | YES | YES | 74.9/173.4 | 79.5/143.4 | YES |
| HOCH₂COOH | | | | | | |
| Maleic | YES | YES | YES | 172.0 | 166.9 | YES |
| Cis-HOOC(CH₂)₄COOH | | | | | | |

The DSC data confirmed the p-toluenesulfonate salt as a conglomerate, the melting point of the racemic salt was lowered by between 27.6 to 28.6 °C (186.6 °C) than that of the enantiomeric salt (see below). Solubility tests in water and methanol showed that the solubility of the racemic p-toluenesulfonate salt (ca. 13 mL/g) was significantly higher than that of the enantiomeric salt (ca. 25 mL/g). Similarly, in methanol the racemic p-toluenesulfonate salt had a solubility of 37 mL/g while the enantiomeric salt was 65 mL/g. The IR match was confirmed.

**Table 5**

| **p-Toluenesulfonate Salt** | **Melting point °C** | **ΔH J/g** |
|---|---|---|
| Racemic | 186.6 | 100.2 |
| (-) enantiomer | 213.2 | 145.6* |
| (+) enantiomer | 214.2 | |

| | | |
|---|---|---|
| * corrected value | | |

The other candidates were recrystallized from methanol/water/IPA and methanol/water mix. The results obtained for the racemic maleic salt are inconclusive. The IR spectrum matched and the melting point difference is significant (see below). The other candidates were confirmed as non-conglomerates.

**Table 6**

| **Maleic Acid Salt** | **Melting point °C** |
|---|---|
| Racemic | 172.3 |
| Single enantiomer | 238.8 |

The preceding examples can be repeated with similar success by substituting the generically or specifically described reactants and/or operating conditions of this invention for those used in the preceding examples.

## Claims

1. A process for the preparation of single enantiomers of a compound of formula (B), in either the *cis* or *trans* configuration, or a pharmaceutically acceptable salt or ester thereof, said process comprising: 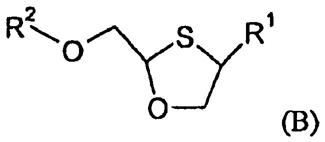 wherein
R¹ is pyrimidine base or a pharmaceutically acceptable derivative thereof;
R² is hydrogen, -C(O)-R³, or together with the oxygen atom to which it is attached forms an ester derived from a polyfunctional acid; and
R³ is hydrogen, straight or branched chain alkyl, alkoxyalkyl, aralkyl, aryloxyalkyl, aryl, substituted dihydropyridinyl, a sulphonate ester, a sulfate ester, an amino acid ester, a mono, di- or triphosphate esters; said process comprising forming a conglomerate salt of racemic mixture or an enantiomerically enriched mixture of a compound of formula (B) with an acid wherein the resulting conglomerate salt has the following characteristics:
the IR spectrum of the salt of the racemic compound, a 1:1 mixture of (-) and (+) crystals, is identical to each of the single enantiomers, and the salt of the racemic compound has a melting point lower than that of either single enantiomer; and
resolving said mixture by crystallization.

2. A process according to claim 1,
wherein the crystallization process is preferential crystallization, entrainment or cyclic entrainment.

3. A process according to claim 1 or 2, wherein
R¹ is selected from the following formulae: 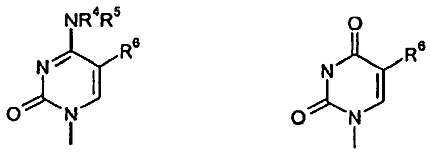 R⁴ and R⁵ are in each case independently H, straight, branched or cyclic C₁₋₆ alkyl, straight, branched or cyclic C₂₋₆ alkenyl, C₆₋₁₄ aryl, or 5-10 membered heteroaromatic ring containing 1-3 heteroatoms selected from O, N, and S; and
R⁶ is hydrogen, hydroxymethyl, trifluoromethyl, straight, branched or cyclic C₁₋₆ alkyl, straight, branched or cyclic C₂₋₆ alkenyl, bromine, chlorine, fluorine, or iodine.

4. A process according to any one of claims 1 to 3, wherein R³ is hydrogen, straight or branched chain alkyl, alkoxyalkyl, aralkyl, aryloxyalkyl, aryl, substituted dihydropyridinyl, alkylsulphonyl, aralkylsulphonyl, a sulfate ester, an amino acid ester, and mono, di- or triphosphate esters, and pharmaceutically acceptable salts and esters thereof, preferably wherein R³ is hydrogen, methyl, ethyl, n-propyl, t-butyl, n-butyl, methoxymethyl, benzyl, phenoxymethyl, phenyl, phenyl substituted by halogen, C₁₋₄ alkyl or C₁₋₄ alkoxy, N-methyldihydropyridinyl, methanesulphonyl, L-valyl or L-isoleucyl,
more preferably, wherein R³ is methyl, ethyl, n-propyl, t-butyl, n-butyl, methoxymethyl, benzyl, phenoxymethyl, phenyl, or phenyl substituted by halogen, C₁₋₄ alkyl or C₁₋₄ alkoxy.

5. A process according to anyone of claims 1 to 4, wherein R² is an ester derived from a dicarboxylic acid of the formula HO₂C(CH₂)nCO₂H where n is an integer of 1 to 10.

6. A process according to anyone of claims 1 to 5, wherein single enantiomers of formula (B) in the *trans* configuration or wherein single enantiomers of formula (B) in the *cis* configuration are prepared.

7. A process according to anyone of claims 1 to 6, wherein the single enantiomers of the conglomerate salt show a much lower solubility than the racemate of the conglomerate salt in polar solvents.

8. A process according to anyone of claims 1 to 7, wherein enantiomer separation of the enantiomeric mixture is performed by seeding a supersaturated solution of the conglomerate salt with the desired single enantiomer.

9. A process according to anyone of claims 1 to 8, wherein R¹ is cytosine or 5-fluorocytosine, preferably wherein R¹ is cytosine, more preferably wherein R¹ is 5-fluorocytosine.

10. A process according to anyone of claims 1 to 9, wherein said acid is hydrochloric acid, hydrobromic acid, sulfuric acid, tetrafluoroboric acid, methanesulfonic acid, benzenesulfonic acid, *para*-toluenesulfonic acid, *p*-amino benzenesulfonic acid, *p*-chloro benzenesulfonic acid, *p-*hydroxy benzenesulfonic acid, chloroacetic acid, dichloroacetic acid, trichloroacetic acid, glycolic acid, pyruvic acid, succinic acid, adipic acid, maleic acid, fumaric acid, citric acid, or a mixture thereof, wherein said acid is *para*-toluenesulfonic acid, maleic acid or a mixture thereof.

11. A process according to claim 1, wherein the conglomerate salt formed is *cis* 2'-deoxy-3'-oxa-4'-thiocytidine, preferably wherein a conglomerate salt of *cis* 2'-deoxy-3'-oxa-4'-thiocytidine is formed in which the single enantiomers show a lower solubility than the racemate in polar solvents, more preferably wherein said conglomerate salt of *cis* 2'-deoxy-3'-oxa-4'-thiocytidine is the *para*-toluenesulfonic acid salt of 2'-deoxy-3'-oxa-4'-thiocytidine having an eutectic point between about 185°C and 187°C or wherein said conglomerate salt of *cis* 2'-deoxy-3'-oxa-4'-thiocytidine is the malic salt of 2'-deoxy-3'-oxa-4'-thiocytidine having an eutectic point between about 171°C and 173°C.

12. A process according to any one of claims 1 to 11 wherein the single enantiomer further comprises a second isomer of a compound of formula (B) in an amount equal to or less than 1%.

13. A process for resolving *cis*-2-hydroxymethyl-4-(cytosin-1'-yl)-1,3-oxathiolane or derivatives or salts thereof comprising:
a) reacting said *cis*-2-hydroxymethyl-4-(cytosin-1'-yl)-1,3-oxathiolane with an achiral acid to produce *cis*-2-hydroxymethyl-4-(cytosin-1'-yl)-1,3-oxathiolane·achiral acid salt;
b) preparing a solution of *cis*-2-hydroxymethyl-4-(cytosin-1'-yl)-1,3-oxathiolane·achiral acid salt having an enantiomeric excess greater than zero;
c) adding to said solution an amount of (+) or (-)*-cis*-2-hydroxymethyl-4-(cytosin-1'-yl)-1,3-oxathiolane_{·}achiral acid salt sufficient to initiate crystallization;
d) recovering substantially one of said (+) or (-)-*cis*-2-hydroxymethyl-4-(cytosin-1'-yl)-1,3-oxathiolane·achiral acid salt; and
e) converting said (+) or (-)-*cis*-2-hydroxymethyl-4-(cytosin-1'-yl)-1,3-oxathiolane·achiral acid salt into said (+) or (-)-*cis*-2-hydroxymethyl-4-(cytosin-1'-yl)-1,3-oxathiolane or salts, preferably wherein said achiral acid is hydrochloric acid, hydrobromic acid, sulfuric acid, tetrafluoroboric acid, methanesulfonic acid, benzenesulfonic acid, *para*-toluenesulfonic acid, *p*-amino benzenesulfonic acid, *p*-chloro benzenesulfonic acid, *p*-hydroxy benzenesulfonic acid, chloroacetic acid, dichloroacetic acid, trichloroacetic acid, glycolic acid, pyruvic acid, succinic acid, adipic acid, maleic acid, fumaric acid, citric acid, or a mixture thereof,
more preferably wherein said acid is para-toluenesulfonic acid.

14. A conglomerate salt of *cis* 2'-deoxy-3'-oxa-4'-thiocytidine which is the para-toluenesulfonic acid salt of 2'-deoxy-3'-oxa-4'-thiocytidine having an eutectic point between about 185°C and 187°C, or the maleic salt of 2'-deoxy-3'-oxa-4'-thiocytidine having an eutectic point between about 171°C and 173°C.

15. A process for the preparation of a single enantiomer, preferably the *cis* configuration, of a compound of formula (B) or a pharmaceutically acceptable salt or ester thereof, wherein the enantiomer comprises methyl tosylate in an amount equal to or less than 2 ppm, the process comprising the steps of:
(a) forming a conglomerate salt of a racemic mixture or an enantiomerically enriched mixture of a compound of formula (B) with a tosic acid, preferably *para*-toluenesulfonic acid,
(b) obtaining an enantiomerically enriched mixture of the salts of the enantiomers by crystallization; and
(c) obtaining the free base of the enantiomerically enriched mixture, preferably wherein the compound of formula (B) is 2'-deoxy-3'-oxa-4'-thiocytidine, and wherein the enantiomer optionally further comprises a second isomer of a compound of formula (B) in an amount equal to or less than 1 %.

## Patentansprüche

1. Verfahren zur Herstellung von einzelnen Entantiomeren einer Verbindung nach Formel (B), entweder in der cis oder trans Konfiguration, oder ein pharmazeutisch akzeptables Salz oder Ester davon, wobei dieses Verfahren umfasst: 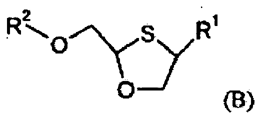 wobei
R1 Pyrimidin Base oder ein pharmazeutisch akzeptables Derivat davon ist; R2 Wasserstoff ist, -C(O)R3 oder zusammen mit dem Sauerstoffatom, zu welchem es gebunden ist, einen Ester bildet, abstammend von polyfunktionaler Säure; und
R3 Wasserstoff, gerade oder verzweigte Alkylkette, Alkoxyalkyl, Aralkyl, Aryloxyalkyl, Aryl, substituiertes Dihydropyridinyl, ein Sulfonsäureester, ein Sulfatester, ein Aminosäureester, ein Mono-, Di- oder Triphosphatester ist; dieser Verfahren umfassend Bilden eines Konglomeratsalzes einer racemischen Mischung oder eine Enantiomer angereicherte Mischung einer Verbindung nach Formel (B) mit einer Säure, wobei das resultierende Konglomeratsalz die folgenden Eigenschaften hat:
das IR Spektrum des Salzes der racemischen Mischung, eine 1:1 Mischung von (-) und (+) Kristallen, ist identisch mit jedem der einzelnen Enantiomere und das Salz der racemischen Verbindung hat einen geringeren Schmelzpunkt als jedes einzelne Enantiomer;
und Lösen dieser Mischung durch Kristallisation.

2. Verfahren nach Anspruch 1,
wobei das Kristallisationsverfahren bevorzugt Kristallisation ist, Entrainment oder zyklisches Entrainment ist.

3. Verfahren nach Anspruch 1 oder 2, wobei R1 aus der folgenden Formel ausgewählt ist: 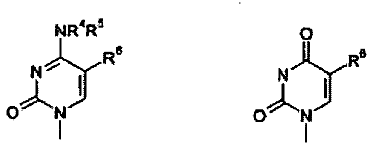 R4 und R5 sind in jedem Fall unabhängig H, gerade, verzweigt oder zyklisches C1-6 Alkyl, gerade, verzweigtes oder zyklisches C2-6 Alkenyl, C6-14 Aryl oder 5-10 gliedriger heteroaromatischer Ring, der 1-3 Heteroatome enthält, ausgewählt aus O, N und S; und
R6 ist Wasserstoff, Hydroxymethyl, Trifluormethyl, gerade, verzweigtes oder zyklisches C1-6 Alkyl, gerade, verzweigtes oder zyklisches C2-6 Alkenyl, Brom, Chlor, Fluor oder Jod.

4. Verfahren nach einem der Ansprüche 1 bis 3, wobei R3 Wasserstoff, gerade oder verzweigtes Kettenalkyl, Alkoxyalkyl, Aralkyl, Aryloxyalkyl, Aryl, substituiertes Dihydropyridinyl, Alkylsulphonyl, Aralkylsulphonyl, ein Sulfatester, ein Aminosäureester, und Mono-, Di- oder Triphosphatester und pharmazeutisch akzeptable Salze oder Ester davon ist, wobei R3 bevorzugt Wasserstoff, Methyl, Ethyl, n-Propyl, t-Butyl, n-Butyl, Methoxymethyl, Benzyl, Phenoxymethyl, Phenyl, Phenyl sustituiert durch Wasserstoff, C1-4 Alkyl oder C1-4 Alkoxy, N-Methyldihydropyridinyl, Methansulphonyl, L-Valyl oder L-Isoleucyl ist, wobei mehr bevorzugt R3 Methyl, Ethyl, n-Propyl, t-Butyl, n-Butyl, Methoxymethyl, Benzyl, Phenoxymethyl, Phenyl, oder Phenyl substituiert mit Halogen, C1-4 Alkyl oder C1-4 Alkoxy ist.

5. Verfahren nach einem der Ansprüche 1 bis 4, wobei R2 ein Ester ist, abgeleitet von einer Dikarbonsäure der Formel HO2C(CH2)nCO2H, wo n eine ganze Zahl von 1 bis 10 ist.

6. Verfahren nach einem der Ansprüche 1 bis 5, wobei einzelne Enantiomere der Formel (B) in der trans Konfiguration oder wobei einzelne Enantiomere der Formel (B) in der cis Konfiguration hergestellt sind.

7. Verfahren nach einem der Ansprüche 1 bis 6, wobei die einzelnen Enantiomere des Konglomeratsalzes eine viel geringere Löslichkeit als die Racemate des Konglomeratsalzes in polaren Lösungsmitteln zeigen.

8. Verfahren nach einem der Ansprüche 1 bis 7, wobei Enantiomere Trennung der enantiomeren Mischung durch Beimpfen einer übersättigten Lösung des Konglomeratsalzes mit dem gewünschten einzelnen Enantiomer durchgeführt wird.

9. Verfahren nach einem der Ansprüche 1 bis 8, wobei R1 Cytosin oder 5-Fluorcytosin ist, wobei bevorzugt R1 Cytosin ist, wobei R1 bevorzugter 5-Fluorcytosin ist.

10. Verfahren nach einem der Ansprüche 1 bis 9, wobei die Säure Salzsäure, Bromwasserstoffsäure, Schwefelsäure, Tetrafluoroborsäure, Methansulfonsäure, Benzolsulfonsäure, para-Toluolsulfonsäure, p-AminoBenzolsulfonsäure, p-chloro Benzolsulfonsäure, p-Hydroxybenzolsulfonsäure, Chloressigsäure, Dichloressigsäure, Trichloressigsäure, Glykolsäure, Brenztraubensäure, Bernsteinsäure, Adipinsäure, Maleinsäure, Fumarsäure, Zitronensäure oder eine Mischung davon ist, wobei die Säure para-Toluolsulfonsäure, Maleinsäure oder eine Mischung davon ist.

11. Verfahren nach Anspruch 1, wobei das gebildete Konglomeratsalz cis 2'-deoxy-3'-oxa-4'-Thiocytidin ist, wobei bevorzugt ein Konglomeratsalz von cis 2'-deoxy-3'-oxa-4'-Thiocytidin gebildet wird, in welchem die einzelnen Enantiomere eine geringere Löslichkeit als das Racemat in polaren Lösungsmitteln zeigt, wobei bevorzugter das Konglomeratsalz von cis 2'-deoxy-3'-oxa-4'-Thiocytidin das para-Toluolsulfonsäuresalz von 2'-deoxy-3'-oxa-4'-Thiocytidin ist, das einen Eutektischen Punkt zwischen ungefähr 185 °C und 187 °C aufweist oder wobei das Konglomeratsalz von cis 2'-deoxy-3'-oxa-4'-Thiocytidin das Maleinsalz von 2'-deoxy-3'-oxa-4'-Thiocytidin ist, das einen Eutektischen Punkt zwischen ungefähr 171 °C und 173 °C aufweist.

12. Verfahren nach einem der Ansprüche 1 bis 11, wobei das einzelne Enantiomer ferner ein zweites Isomer einer Verbindung von Formel (B) in einem Anteil gleich oder weniger als 1 % umfaßt.

13. Verfahren zum Trennen von cis-2-Hydroxymethyl-4-(Cytosin-1'-yl)-1,3-Oxathiolan oder Derivate oder Salze davon, umfassend:
a) Reagieren des cis-2-Hydroxymethyl-4-(Cytosin-1'-yl)-1,3-Oxathiolan mit einer achiralen Säure, um cis-2-Hydroxymethyl-4-(Cytosin-l'-yl)-1,3-Oxathiolan•achirales Säuresalz zu bilden;
b) Zubereiten einer Lösung von cis-2-Hydroxymethyl-4-(Cytosin-1'-yl)-1,3-Oxathiolan achirales Säuresalz, das einen enantiomeren Überschuß größer als Null aufweist;
c) Hinzufügen eines Anteils von (+) oder (-)-cis-2-Hydroxymethyl-4-(Cytosin-1'-yl)-1,3-Oxathiolan•achirales Säuresalz zu der Lösung, ausreichend um Kristallisation zu starten;
d) erhebliches Wiedergewinnen eines des (+) oder (-)-cis-2-Hydroxymethyl-4-(Cytosin-1'-yl)-1,3-Oxathiolan achiralen Säuresalzes; und
e) Umwandeln des (+) oder (-)-cis-2-Hydroxymethyl-4-(Cytosin-1'-yl)-1,3-e) Oxathiolan achiralen Säuresalzes in das cis-2-Hydroxymethyl-4-(Cytosin-1'-yl)-1,3-Oxathiolan oder Salz, wobei bevorzugt die achirale Säure Salzsäure, Bromwasserstoffsäure, Schwefelsäure, Tetrafluorborsäure, Methansulfonsäure, Benzolsulfonsäure, para-Toluolsulfonsäure, p-Aminobenzolsulfonsäure, p-chloro Benzolsulfonsäure, p-Hydroxybenzolsulfonsäure, Chloressigsäure, Dichloressigsäure, Trichloressigsäure, Glykolsäure, Brenztraubensäure, Bernsteinsäure, Adipinsäure, Maleinsäure, Fumarsäure, Zitronensäure oder eine Mischung davon ist, wobei bevorzugter die Säure para-Toluolsulfonsäure ist.

14. Konglomeratsalz von cis 2'-deoxy-3'-oxa-4'-Thiocytidin, welches das para-Toluolsulfonsäuresalz von 2'-deoxy-3'-oxa-4'-Thiocytidin ist, das einen Eutektischen Punkt zwischen ungefähr 185 °C und 187 °C aufweist oder das Maleinsalz von 2'-deoxy-3'-oxa-4'-Thiocytidin einen Eutektischen Punkt zwischen ungefähr 171 °C und 173 °C aufweist.

15. Verfahren zur Herstellung eines einzelnen Enantiomers, bevorzugt der cis Konfiguration, einer Verbindung nach Formel (B) oder eines pharmazeutisch akzeptablen Salzes oder Esters davon, wobei das Enantiomer Methyltosylat in einem Anteil gleich oder weniger als 2 ppm umfaßt, der Verfahren die Schritte umfassend:
a) Bilden eines Konglomeratsalzes einer racemischen Mischung oder einer enantiomer angereicherten Mischung einer Verbindung nach Formel (B) mit einer Tosinsäure, bevorzugt para-Toluolsulfonsäure,
b) Erhaltend einer Enantiomer angereicherten Mischung des Salzes der Enantiomere durch Kristallisation; und
c) Erhaltend die freien Basen der Enantiomer angereicherten Mischung, wobei bevorzugt die Verbindung nach Formel (B) 2'-deoxy-3'-oxa-4'-Thiocytidin ist und wobei das Enantiomer gegebenenfalls ferner ein zweites Isomer einer Verbindung nach Formel (B) in einer Menge gleich oder weniger als 1 % umfaßt.

## Revendications

1. Procédé pour la préparation d'énantiomères uniques d'un composé de formule (B), en configuration *cis* ou *trans,* ou d'un sel ou d'un ester pharmaceutiquement acceptable de celui-ci, ledit procédé comprenant : 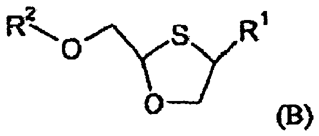 dans lequel
R¹ est la base pyrimidine ou un dérivé pharmaceutiquement acceptable de celle-ci ;
R² est hydrogène, -C(O)-R³, ou conjointement avec l'atome d'oxygène auquel il est attaché, forme un ester dérivé d'un acide polyfonctionnel ; et
R³ est hydrogène, alkyle à chaîne droite ou ramifiée, alcoxyalkyle, aralkyle, aryloxyalkyle, aryle, dihydropyridinyle substitué, un ester de sulfonate, un ester de sulfate, un ester d'acide aminé, des esters de mono, di ou triphosphate ;
ledit procédé comprenant la formation d'un sel de conglomérat de mélange racémique ou un mélange énantiomériquement enrichi d'un composé de formule (B) avec un acide où le sel de conglomérat résultant a les caractéristiques suivantes :
le spectre IR du sel du composé racémique, un mélange 1:1 de cristaux (-) et (+), est identique à chacun des énantiomères uniques, et le sel du composé racémique a un point de fusion inférieur à celui de n'importe quel énantiomère unique ; et
la résolution dudit mélange par cristallisation.

2. Procédé selon la revendication 1,
dans lequel le procédé de cristallisation est la cristallisation préférentielle, l'entrainement ou l'entrainement cyclique.

3. Procédé selon la revendication 1 ou 2, dans lequel
R¹ est sélectionné parmi les formules suivantes : 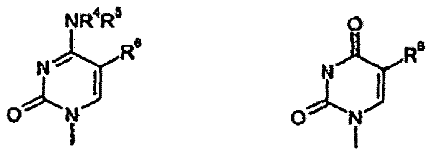 R⁴ et R⁵ sont dans chaque cas indépendamment H, alkyle en C₁₋₆ à chaîne droite, ramifiée ou cyclique, alcényle en C₂₋₆ à chaîne droite, ramifiée ou cyclique, aryle en C₆₋₁₄, ou cycle hétéroaromatique de 5-10 chaînons contenant 1-3 hétéroatomes sélectionnés parmi O, N, et S ; et
R⁶ est hydrogène, hydroxyméthyle, trifluorométhyle, alkyle en C₁₋₆ à chaîne droite, ramifiée ou cyclique, alcényle en C₂₋₆ à chaîne droite, ramifiée ou cyclique, brome, chlore, fluor ou iode.

4. Procédé selon l'une quelconque des revendications 1 à 3, dans lequel R³ est hydrogène, alkyle à chaîne droite ou ramifiée, alcoxyalkyle, aralkyle, aryloxyalkyle, aryle, dihydropyridinyle substitué, alkylsulfonyle, aralkylsulfonyle, un ester de sulfate, un ester d'acide aminé et des esters de mono, di ou triphosphate et les sels et esters pharmaceutiquement acceptables de ceux-ci, de préférence dans lequel R³ est hydrogène, méthyle, éthyle, n-propyle, t-butyle, n-butyle, méthoxyméthyle, benzyle, phénoxyméthyle, phényle, phényle substitué par halogène, alkyle en C₁₋₄ ou alcoxy en C₁₋₄, N-méthyldihydropyridinyle, méthanesulfonyle, L-valyle ou L-isoleucyle, davantage préféré, dans lequel R³ est méthyle, éthyle, n-propyle, t-butyle, n-butyle, méthoxyméthyle, benzyle, phénoxyméthyle, phényle, ou phényle substitué par halogène, alkyle en C₁₋₄ ou alcoxy en C₁₋₄.

5. Procédé selon l'une quelconque des revendications 1 à 4, dans lequel R² est un ester dérivé d'un acide dicarboxylique de formule HO₂C(CH₂)ₙCO₂H où n est un entier de 1 à 10.

6. Procédé selon l'une quelconque des revendications 1 à 5, dans lequel les énantiomères uniques de formule (B) en configuration *trans* ou dans lequel les énantiomères uniques de formule (B) en configuration *cis* sont préparés.

7. Procédé selon l'une quelconque des revendications 1 à 6, dans lequel les énantiomères uniques du sel de conglomérat montrent une solubilité beaucoup plus faible que le racémate du sel de conglomérat dans des solvants polaires.

8. Procédé selon l'une quelconque des revendications 1 à 7, dans lequel la séparation énantiomérique du mélange énantiomérique est effectuée par ensemencement d'une solution sursaturée du sel de conglomérat avec l'énantiomère unique souhaité.

9. Procédé selon l'une quelconque des revendications 1 à 8, dans lequel R¹ est la cytosine ou la 5-fluorocytosine, de préférence dans lequel R¹ est la cytosine, davantage préféré dans lequel R¹ est la 5-fluorocytosine.

10. Procédé selon l'une quelconque des revendications 1 à 9, dans lequel ledit acide est l'acide chlorhydrique, l'acide bromhydrique, l'acide sulfurique, l'acide tétrafluoroborique, l'acide méthanesulfonique, l'acide benzènesulfonique, l'acide *para*-toluènesulfonique, l'acide *p*-aminobenzènesulfonique, l'acide *p*-chlorobenzènesulfonique, l'acide *p*-hydroxybenzènesulfonique, l'acide chloroacétique, l'acide dichloroacétique, l'acide trichloroacétique, l'acide glycolique, l'acide pyruvique, l'acide succinique, l'acide adipique, l'acide maléique, l'acide fumarique, l'acide citrique ou un mélange de ceux-ci, dans lequel ledit acide est l'acide *para-*toluènesulfonique, l'acide maléique ou un mélange de ceux-ci.

11. Procédé selon la revendication 1, dans lequel le sel de conglomérat formé est la *cis* 2'-déoxy-3'-oxa-4'-thiocytidine, de préférence dans lequel un sel de conglomérat de *cis* 2'-déoxy-3'-oxa-4'-thiocytidine est formé dans lequel les énantiomères uniques montrent une solubilité plus faible que celle du racémate dans des solvants polaires, davantage préféré dans lequel ledit sel de conglomérat de cis 2'-déoxy-3'-oxa-4'-thiocytidine est le sel de l'acide *para*-toluènesulfonique de 2'-déoxy-3'-oxa-4'-thiocytidine ayant un point eutectique entre environ 185°C et 187°C, ou dans lequel le sel de conglomérat de la *cis* 2'-déoxy-3'-oxa-4'-thiocytidine est le sel malique de la 2'-déoxy-3'-oxa-4'-thiocytidine ayant un point eutectique entre environ 171°C et 173°C.

12. Procédé selon l'une quelconque des revendications 1 à 11, dans lequel l'énantiomère unique comprend en plus un second isomère d'un composé de formule (B) dans une quantité égale ou inférieure à 1%.

13. Procédé pour la résolution de *cis*-2-hydroxyméthyl-4-(cytosin-1'-yl)-1,3-oxathiolane ou des dérivés ou des sels de celui-ci comprenant :
a) la réaction dudit *cis*-2-hydroxyméthyl-4-(cytosin-1'-yl)-1,3-oxathiolane avec un acide achiral pour produire le sel d'acide achiral de *cis*-2-hydroxyméthyl-4-(cytosin-1'-yl)-1,3-oxathiolane ;
b) la préparation d'une solution de sel d'acide achiral de *cis*-2-hydroxyméthyl-4-(cytosin-1'-yl)-1,3-oxathiolane ayant un excès énantiomérique supérieur à zéro ;
c) l'addition à ladite solution d'une quantité de sel d'acide achiral de (+) ou (-)-*cis*-2-hydroxyméthyl-4-(cytosin-1'-yl)-1,3-oxathiolane suffisante pour initier la cristallisation ;
d) la récupération sensiblement de l'un desdits sels d'acide achiral de (+) ou (-)-cis-2-hydroxyméthyl-4-(cytosin-1'-yl)-1,3-oxathiolane ; et
e) la conversion dudit sel d'acide achiral de (+) ou (-)-*cis*-2-hydroxyméthyl-4-(cytosin-1'-yl)-1,3-oxathiolane en ledit (+) ou (-)-*cis*-2-hydroxyméthyl-4-(cytosin-1'-yl)-1,3-oxathiolane ou ses sels, de préférence dans lequel ledit acide achiral est l'acide chlorhydrique, l'acide bromhydrique, l'acide sulfurique, l'acide tétrafluoroborique, l'acide méthanesulfonique, l'acide benzènesulfonique, l'acide *para-*toluènesulfonique, l'acide *p*-aminobenzènesulfonique, l'acide *p*-chlorobenzènesulfonique, l'acide *p*-hydroxybenzènesulfonique, l'acide chloroacétique, l'acide dichloroacétique, l'acide trichloroacétique, l'acide glycolique, l'acide pyruvique, l'acide succinique, l'acide adipique, l'acide maléique, l'acide fumarique, l'acide citrique ou un mélange de ceux-ci,
davantage préféré dans lequel ledit acide est l'acide *para*-toluènesulfonique.

14. Sel de conglomérat de la *cis* 2'-déoxy-3'-oxa-4'-thiocytidine qui est le sel d'acide *para*-toluènesulfonique de la 2'-déoxy-3'-oxa-4'-thiocytidine ayant un point eutectique entre environ 185°C et 187°C, ou le sel maléique de la 2'-déoxy-3'-oxa-4'-thiocytidine ayant un point eutectique entre environ 171°C et 173°C.

15. Procédé pour la préparation d'un énantiomère unique, de préférence en configuration *cis,* d'un composé de formule (B) ou d'un sel ou ester pharmaceutiquement acceptable de celui-ci, dans lequel l'énantiomère comprend du tosylate de méthyle dans une quantité égale ou inférieure à 2 ppm, le procédé comprenant les étapes de :
(a) formation d'un sel de conglomérat d'un mélange racémique ou d'un mélange énantiomériquement enrichi d'un composé de formule (B) avec un acide tosylique, de préférence de l'acide *para*-toluènesulfonique ;
(b) obtention d'un mélange énantiomériquement enrichi des sels des énantiomères par cristallisation ; et
(c) obtention de la base libre du mélange énantiomériquement enrichi, de préférence dans lequel le composé de formule (B) est le 2'-déoxy-3'-oxa-4'-thiocytidine, et dans lequel l'énantiomère facultativement comprend en plus un second isomère d'un composé de formule (B) dans une quantité égale ou inférieure à 1%.
